# EUROPEAN PATENT APPLICATION

(11) **EP 4 019 060 A1**
(43) Date of publication of application: **29.06.2022**
(21) Application number: 21208955.1
(22) Date of filing: 18.11.2021
(51) Int. Cl.: A61M 1/00, A61M 16/20, A61M 39/22, A61M 16/04

(54) **CLOSED SUCTION CATHETER CONTROLLER**

(30) Priority: 22.12.2020 TW 109216958 U
(71) Applicant: Sunder Biomedical Tech. Co., Ltd., Yunlin County 634 (TW)
(72) Inventor: LIAO, Yuan-Ting, 634 Yunlin County (TW); LIAO, Chi-Chao, 634 Yunlin County (TW); HUANG, Wei-Feng, 634 Yunlin County (TW)
(74) Representative: Straus, Alexander

(57) **Abstract**

A closed suction catheter controller (10) includes a valve seat (20) and a movable valve (30). The valve seat (20) includes a body (22), a first vent tube (24) and a second vent tube (26). The body (22) has an accommodating space (221), a first opening (222), a second opening (223) and a third opening (224). The movable valve (30) is rotatably and axially movable in the valve seat (20), and has a plug (36) accommodated in the accommodating space (221). The plug (36) has a convex circular arc surface (361). When the movable valve (30) is in the sealing position, the first opening (222) and the second opening (223) are closed by the convex circular arc surface (361). When the movable valve (30) is in the venting position, the convex circular arc surface (361) is separated from the first opening (222) and the second opening (223). In this way, the present invention uses the convex circular arc surface (361) on the plug (36) to form an outwardly protruding arc slope to seal the first opening (222) and the second opening (223), which is more structurally fit, and further improves the sealing effect.

## Description

### BACKGROUND OF THE INVENTION

### 1. Field of the Invention

The invention is a medical device related to the patient's respiratory system, in particular to a closed suction catheter controller.

### 2. Description of the Related Art

For the removal of sputum from the respiratory tract of severely ill patients, two methods are usually used in clinical practice: open suction catheter and closed suction catheter.

When the open suction catheter is in use, the patient's ventilator needs to be turned off. Since the suction process is open, in addition to bringing bacteria in the air into the patient's respiratory tract, causing unnecessary infections, the patient may also produce droplets due to coughing during aspiration, and even spray sputum on the nurses, clothes or beds, which increases the risk of infections of the nurses and other patients. Because the closed suction catheter is used to slip on the respirator, there is no need to close the respirator when performing sputum suction, and it is used in a closed environment throughout the process, effectively avoiding the scattering of the patient's mouth foam and sputum. It has greatly increased the safety of medical staff and at the same time reduced the risk of spreading infection, and it has gradually become a frequently used sputum suction device in clinical practice.

However, the aforementioned closed suction catheter relies on the assistance of the suction device and the pressure control of the controller to suck sputum during suction. Many closed suction catheters on the market have a problem of poor suction due to the poor airtightness of the controller, so there is a need for improvement.

### SUMMARY OF THE INVENTION

The present invention has been accomplished under the circumstances in view. It is therefore the main object to provide a closed suction catheter controller, which has better sealing properties.

To achieve this and other objects of the present invention, a closed suction catheter controller provided by the present invention comprises a valve seat and a movable valve. The valve seat comprises a body, a first vent tube and a second vent tube. The body comprises an accommodating space, a first opening and a second opening radially and oppositely communicating with the accommodating space, and a third opening axially communicating with the accommodating space. The first vent tube has one end thereof connected to the body and disposed in communication with the first opening. The second vent tube has one end thereof connected to the body and disposed in communication with the second opening. The movable valve is rotatably placed in the valve seat and axially movable relative to the valve seat between a sealing position and a venting position. The movable valve comprises a plug accommodated in the accommodating space. The plug comprises a convex circular arc surface. When the movable valve is in the sealing position, the first opening and the second opening are closed by the convex circular arc surface. When the movable valve is in the venting position, the convex circular arc surface is separated from the first opening and the second opening.

In this way, the present invention uses the convex circular arc surface on the plug to form an outwardly protruding arc slope to seal the first opening and the second opening, which is more structurally fit, and further improves the sealing effect.

### BRIEF DESCRIPTION OF THE DRAWINGS

FIG. 1 is an oblique top elevational view of a closed suction catheter controller in accordance with the present invention.
FIG. 2 is an exploded view of the closed suction catheter controller in accordance with the present invention.
FIG. 3 is a sectional side view of the closed suction catheter controller, which mainly shows the internal structure of the movable valve in the locked position and the sealing position.
FIG. 4 is similar to FIG. 3, mainly showing the internal structure of the movable valve in the movable position and the venting position.
FIG. 5 is a top view of the body in the closed suction catheter controller, which mainly shows the position diagram of the first limiting member set in the body.
FIG. 6 is a top view of the upper cover in the closed suction catheter controller, which mainly shows the position diagram of the second limiting member set in the upper cover.

### DETAILED DESCRIPTION OF THE INVENTION

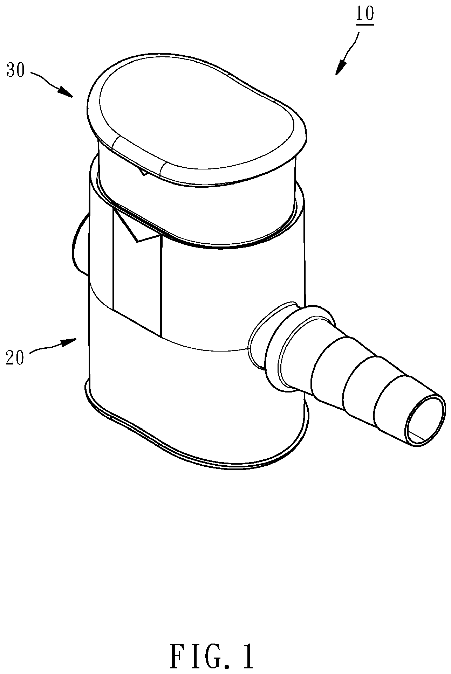

Referring to FIGS. 1-3, a closed suction catheter controller **10** provided by the present invention comprises a valve seat **20** and a movable valve **30.**

The valve seat **20** comprises a body **22,** a first vent tube **24,** a second vent tube **26,** an elastic member **27** and a bottom cover **28.** The body **22** comprises an accommodating space **221,** a first opening **222** and a second opening **223** radially and oppositely communicating with the accommodating space **221,** a third opening **225** and a fourth opening **226** axially and oppositely communicating with the accommodating space **224,** and at least one first limiting member **227.** One end of the first vent tube **24** is connected to the body **22** and communicating with the first opening **222.** One end of the second vent tube **26** is connected to the body **22** and communicating with the second opening **223.** The elastic member **27** is located in the accommodating space **221,** and the bottom cover **28** closes the fourth opening **226.**

It should be noted that the so-called axial direction refers to the vertical direction according to the elevational view shown in FIG. 1, which is also the up and down direction when the closed suction catheter controller **10** provided by the present invention is actually used; the so-called radial direction refers to the horizontal direction according to the elevational view shown in FIG. 1, and is also the front, back, left, and right directions when the closed suction catheter controller **10** provided by the present invention is actually used.

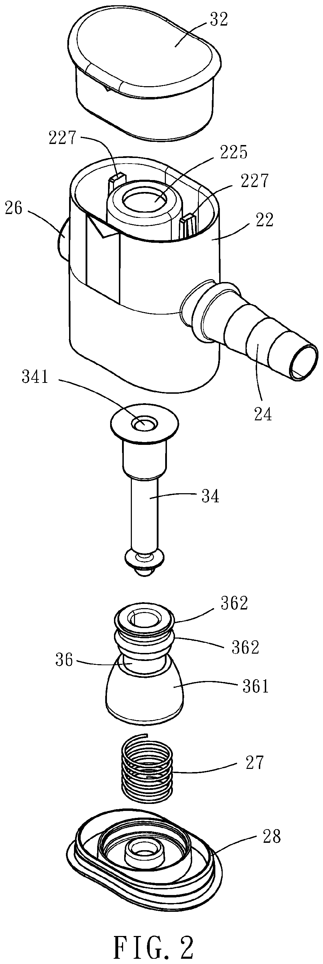

The movable valve **30** is rotatably and axially movably placed in the valve seat **20,** and comprises an upper cover **32,** a shaft **34** and a plug **36.** The upper cover **32** further comprises at least one second limiting member **321.** The second limiting member **321** protrudes axially from the upper cover **32** toward the body **22.** One end of the shaft **34** is connected to the upper cover **32,** and the other end of the shaft **34** passes through the third opening **225** and is located in the accommodating space **221.** The plug **36** is attached onto the part of shaft **34** that is located in the accommodating space **221.** The plug **36** has a convex circular arc surface **361** and at least one sealing ring **362.** The two opposite ends of the elastic member **27** are respectively stopped against the shaft **34** and the bottom cover **28.** As shown in FIGS. 2 to 4, the plug **36** provided in this embodiment is an elastic element made of rubber or silicone. The end of the plug **36** away from the upper cover **32** protrudes into a circular arc cone, and the convex circular arc surface **361** is the side wall surface located on this structure. The structure of the profile of this convex circular arc surface **361** is a protruding circular arc inclined surface, rather than a straight inclined surface. At the other end of plug **36** near the upper cover **32,** there are two sealing rings **362** with sharp corners. Through the push of elastic member **27,** the plug **36** can move axially along with the upper cover **32** and the shaft **34** and move in the accommodating space **221.**

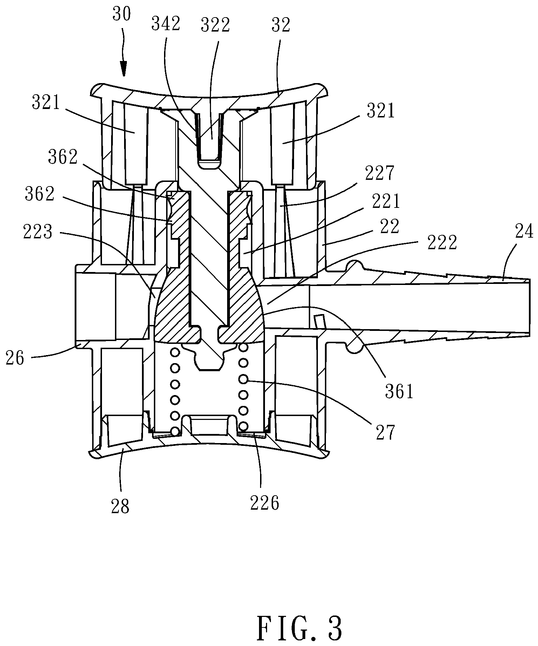

In a preferred embodiment, as shown in FIGS. 3 to 6, the upper cover **32** further has a fixing rod **322,** which protrudes from the upper cover **32** toward the body **22** axially, and at least one groove **323** is longitudinally provided on the fixed rod **322.** One end of the shaft **34** connected to the upper cover **32** is recessed to form a connecting hole **341,** and at least one rib **342** is longitudinally provided in the connecting hole **341.** The fixing rod **322** is arranged in the connecting hole **341** and each rib **342** is engaged with one respective groove **323,** thereby further enhancing the stability of the connection between the upper cover **32** and the shaft **34** and the transmission of rotational force.

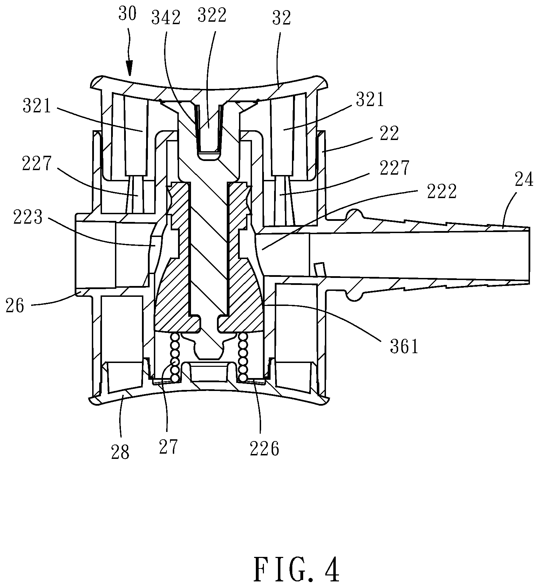

As shown in FIG. 3, when the movable valve **3**0 is in the sealing position, the first opening **222** and the second opening **223** are closed by the convex circular arc surface **361,** so that the first vent tube **24** and the second vent tube **26** cannot be connected, and the sealing rings **362** also happens to seal the third opening **225.** As shown in FIG. 4, when the movable valve **30** is in the venting position, the convex circular arc surface **361** is separated from the first opening **222** and the second opening **223,** so that the first vent tube **24** and the second vent tube **26** are connected to each other, and the airflow can pass.

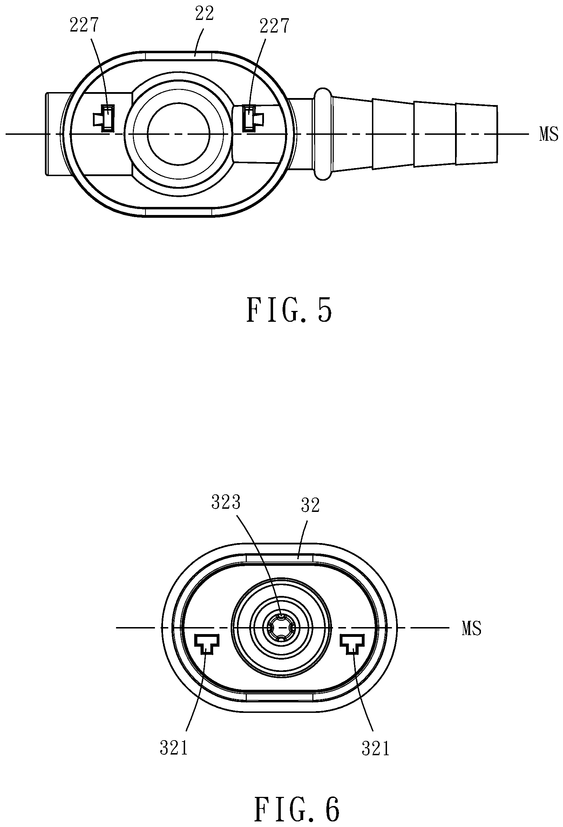

As shown in FIGS. 3 to 6, this embodiment has two first limiting members **227** and two second limiting members **321.** The two first limiting members **227** are set on the same side of the body **22,** and the two second limiting members **321** are set on the same side of the upper cover **32.** As shown in FIGS. 5 and 6, the two first limiting members **227** are the same settings as the two second limiting members **321** or staggered relative to the two second limiting members **321.** The so-called the same settings means that the two first limiting members **227** are located on two corresponding positions of a mirror surface **MS** relative to the second limiting members **321,** as shown in FIG. 3. Staggered refers to staggered on the two relative positions of the mirrored surface **MS,** as shown in FIG. 4. When the movable valve **30** is in the locked position, the two first limiting members **227** and the two second limiting members **321** are in the corresponding positions, so the two first limiting members **227** are against the second limiting members **321,** so that the upper cover **32** cannot perform axial displacement relative to the body **22,** as shown in FIG. 3. When the movable valve **30** rotates 180 degrees from the locked position and is in the movable position, the two first limiting members **227** and the two second limiting members **321** are in relative positions. At this time, the two first limiting members **227** and the two second limiting members **321** no longer no longer abut each other, so that the cover **32** can be axially displaced relative to the body **22,** as shown in FIG. 4.

It should be noted that, in this embodiment, the body **22** and the upper cover **32** are designed as corresponding elliptical shapes, and the movable valve **30** needs to be rotated 180 degrees from the locked position to the movable position. This design has better foolproof effect. However, in other embodiments, the body **22** and the upper cover **32** can be circular or other shaped structures, and the angle at which the movable valve **30** rotates from the locked position to the movable position can be, for example, 90 degrees or other angles, which can also achieve the desired effect of the present invention. Furthermore, the number of the first limiting member **227** and the second limiting member **321** is not limited to two, one or more than two can also achieve the effect of locking and activity.

When the present invention is used clinically, the first vent tube **24** is connected to the aspirator, and the second vent tube **26** is connected to the closed suction catheter and the tracheostomy tube. When the user is not in use, he can turn the movable valve **30** to the locked position and the sealed position. At this time, the closed suction catheter will not be attractive, and there is no need to worry about other people accidentally pressing the controller. When the user uses it, first turn the movable valve **30** to the movable position, and then use the pressing method to axially move the movable valve **30** from the sealing position to the venting position, which will cause the closed suction catheter to produce suction for sputum suction work.

Based on the above characteristics, the closed suction catheter controller **10** provided by the present invention uses the convex circular arc surface **361** on the plug **36** to form an outwardly protruding arc slope to seal the first opening **222** and the second opening **223.** The better fit in the structure further improves the sealing effect. Moreover, through the setting of the first limiting member **227** and the second limiting member **321,** the effect of foolproof and locking is achieved, and the safety of use is also effectively improved.

It must be noted here that the detailed description of the above in conjunction with the drawings is only to provide an embodiment for the purpose of illustrating the technical content and features of the present invention. Any person with general knowledge in the field of the present invention, after understanding the technical content and features of the present invention, without violating the spirit of the present invention, makes various simple modifications, replacements or reductions of components, all should fall within the scope of the patent application disclosed in the present invention.

## Claims

1. A closed suction catheter controller (10), being **characterized in that** the closed suction catheter controller (10) comprising:
a valve seat (20) comprising a body (22), a first vent tube (24) and a second vent tube (26), said body (22) comprising an accommodating space (221), a first opening (222) and a second opening (223) radially and oppositely communicating with said accommodating space (221) and a third opening (225) axially communicating with said accommodating space (221), said first vent tube (24) having one end thereof connected to said body (22) and disposed in communication with said first opening (222), said second vent tube (24) having one end thereof connected to said body (22) and disposed in communication with said second opening (223); and
a movable valve (30) rotatably placed in said valve seat (20) and axially movable relative to said valve seat (20), said movable valve (30) comprising a plug (36) accommodated in said accommodating space (221), said plug (36) comprising a convex circular arc surface (361);
wherein when said movable valve (30) is in a sealing position, said first opening (222) and said second opening (223) are closed by said convex circular arc surface (361), and when said movable valve (30) is in a venting position, said convex circular arc surface (361) is separated from said first opening (222) and said second opening (223).

2. The closed suction catheter controller (10) as claimed in claim 1, wherein said body (22) further comprises an axially arranged fourth opening (226) disposed in communication with said accommodating space (221); said valve seat (30) further comprises a bottom cover (28), said bottom cover (28) closing said fourth opening (226).

3. The closed suction catheter controller (10) as claimed in claim 1, wherein said movable valve (30) further comprises an upper cover (32) and a shaft (34), said shaft (34) having one end thereof connected to said upper cover (32) and an opposite end thereof passing through said third opening (225) and located in said accommodating space (221); said plug (32) is attached onto the part of said shaft (34) where located in said accommodating space (221).

4. The closed suction catheter controller (10) as claimed in claim 3, wherein said body (22) further comprises at least one first limiting member (227) protruded axially from said body (22) toward said upper cover (32); said upper cover (32) comprises at least one second limiting member (321) protruded axially from said upper cover (32) toward said body (22); when said movable valve (30) is in a locked position, said at least one first limiting member (227) abuts said at least one second limiting member (321); when said movable valve (30) is in a movable position, said at least one first limiting member (227) is separated from said at least one second limiting member (321).

5. The closed suction catheter controller (10) as claimed in claim 3, wherein said body (22) further comprises two first limiting members (227) arranged on the same side and protruded axially from said body (22) toward said upper cover (32); said upper cover (32) comprises two second limiting members (321) arranged on the same side and protruded axially from said upper cover (32) toward said body (22); each said first limiting member (227) is the same or staggered setting relative to each said second limiting member (321); when said movable valve (30) is in a locked position, each said first limiting member (227) abuts one respective said second limiting member (321); when said movable valve (30) is in a movable position, each said first limiting member (227) is separated from the respective said second limiting member (321).

6. The closed suction catheter controller (10) as claimed in claim 4 or 5, wherein said movable valve (30) rotates 180 degrees from said locked position to be said movable position.

7. The closed suction catheter controller (10) as claimed in claim 1, wherein said plug (36) further comprises at least one sealing ring (362), and said at least one sealing ring (362) seals said third opening (225) when said movable valve (30) is in said sealing position.

8. The closed suction catheter controller (10) as claimed in claim 1, wherein said upper (32) cover further comprises a fixing rod (322) axially protruded from said upper cover (32) toward said body (22), said fixing rod (322) being longitudinally provided with at least one groove (323); said shaft (34) comprises a connecting hole (341) located on the one end thereof that is connected to said upper cover (32) and at least one rib (342) longitudinally provided in said connecting hole (341), said connecting hole (341) receiving said fixing rod (322), said at least one rib (342) being correspondingly embedded in said at least one groove (323).
